# EUROPEAN PATENT SPECIFICATION

(11) **EP 2 964 741 B1**
(45) Date of publication and mention of the grant of the patent: **06.03.2019**
(21) Application number: 14717195.3
(22) Date of filing: 07.03.2014
(51) Int. Cl.: C11D 1/83, C07C 303/24, C07C 43/11, C07C 305/10, C11D 17/04

(54) **A METHOD OF PREPARING ANHYDROUS ALKYL (ETHOXY) SULPHATE COMPOSITIONS**
VERFAHREN ZUR HERSTELLUNG VON WASSERFREIEN ALKY(ETHOXY)SULFATZUSAMMENSETZUNGEN
PROCÉDÉ DE PRÉPARATION DE COMPOSITIONS ANHYDRES D'ALKYL(ÉTHOXY)SULFATE

(30) Priority: 08.03.2013 IT TO20130186
(43) Date of publication of application: 13.01.2016
(73) Proprietor: Zschimmer & Schwarz Italiana S.p.A., 13038 Tricerro (Vercelli) (IT)
(72) Inventor: GUALA, Fabrizio, I-13039 Trino (Vercelli) (IT); MERLO, Elisabetta, I-13039 Trino (Vercelli) (IT)
(74) Representative: Comoglio, Elena
(86) International application number: PCT/IB2014/059520
(87) International publication number: WO 2014/136091

(56) References cited:
- EP-A- 0 609 574
- EP-A1- 1 059 350
- WO-A1-92/06952
- DE-A1- 19 646 587
- US-A1- 2012 135 910

## Description

The present invention falls within the field of detergent preparations, particularly detergents for washing machines.

The current detergents market, particularly those for washing machines, contemplates more and more the use of concentrated products. The transported water actually represents a high cost, both at the level of raw materials and of finished products. It is also important to reduce the packaging, not only for economic and environmental reasons, but also for the convenience of the consumer who must carry the product home. With a concentrated formulation characterized by reduced packaging, the consumer has the advantage of a product that takes up less space at home and fewer purchases at the supermarket, and in addition it does not soil either the person or the surrounding environment.

In such a perspective, the so-called "ecodoses" were generated, that is 25g or 35g pre-dosed products for single washes contained in suitable water soluble capsules. This way, the storage space is decreased and the product dosage is standard. This results in a lower consumption of packaging plastic, a lower consumption of energy (cold-active products), a lower consumption of water for manufacture, and a lower production of CO₂ for transport.

However, the manufacture of the so-called "ecodoses" requires overcoming the following technical problem. The polyvinyl alcohol that generally forms the capsule film is water soluble: accordingly, the surfactants used in the ecodose must substantially be anhydrous, i.e. they must contain an extremely low percentage of water, bearing in mind that the polyvinyl film of the capsule generally does not tolerate percentages of water higher than 10%. Classical washing products generally have approximately a 1:1:1 ratio of anionic surfactants (such as for example sodium dodecylbenzenesulphonate or sodium lauryl/lauryl ethoxy sulphate), ethoxylated fatty alcohols and soaps that, in addition to washing, restrain the foam. The ethoxylated fatty alcohols are anhydrous and as such suitable for use in ecodoses. Soaps can be easily manufactured with glycol or glycerine as the solvent instead of water and with organic amines as neutralising agents; therefore they are also suitable for use in ecodoses. The problem primarily arises with anionic surfactants, and particularly the alkyl/alkyl ethoxy sulphates. These are classically produced in fact through a sulfation reaction where water is used as the solvent. Such a classical sulfation reaction for the production of neutralized alkyl sulphates / alkyl ethoxy sulphates starting from fatty alcohols/ethoxylated fatty alcohols, which generally is performed in a multitube reactor, may be schematized as follows:
S + O₂ → SO₂ (sulphur burns in the presence of air, generating sulphurous anhydride)
SO₂ + ½O₂→ SO₃ (sulphurous anhydride turns into sulphuric anhydride in the presence of catalysts)
SO₃ + ROH → ROSO₃H (sulphuric anhydride reacts with the fatty alcohol, generating an acidic intermediate)
ROSO₃H + Base → ROSO₃⁻ Base⁺ + H₂O (the acidic intermediate is neutralized with appropriate bases dissolved in an aqueous solvent, generally a NaOH or NH₃ aqueous solution)

EP 1059350 discloses a capsule containing an amine salt of alkyl ether sulfate, a nonionic ethoxylated fatty acid alcohol and etanol amine. Water soluble packages containing alkyl ether sulfates are also disclosed in US 2012/135910. Further processes for making alkyl (ether) sulphates are disclosed in DE 19646587 A1 and WO92/06952.

The problem of the presence of water in the final sulfation reaction product is addressed by the present invention, which provides a method of preparation of neutralized alkyl sulphates and/or alkyl ethoxy sulphates, comprising the steps of:
(a) mixing an ethoxylated fatty alcohol of the formula:

   R'-(O-CH₂-CH₂)ₓ-OH

   wherein R' is a saturated or unsaturated, linear or branched C₆-C₁₆ alkyl, and x is an integer comprised within the range from 1 to 12,
   with an organic amine base; and
(b) adding to the mixture from the preceding step a sulphuric acid ester of the formula:

   R-(O-CH₂-CH₂)ₘ-OSO₃⁻H⁺

   wherein R is a mixture of saturated or unsaturated, linear or branched C₆-C₂₂ alkyls, wherein the amount of C₈ and C₁₀ alkyls is from 1% and 10% and m is 0,
   in order to obtain a substantially anhydrous composition of alkyl sulphates or alkyl ethoxy sulphates neutralized with an organic amine in an ethoxylated fatty alcohol as the solvent.

Advantageously, the method of the invention comprises the use of an ethoxylated fatty alcohol as the solvent instead of water, which allows to obtain an anhydrous or substantially anhydrous final product, which is thus particularly well suited to be used as a component of "ecodoses". Moreover, it should be pointed out that the ethoxylated fatty alcohols used as reaction solvents are commonly used per se as ingredients for laundry detergents, thus the method of the invention allows to obtain a final anhydrous concentrated composition already containing some of the main ingredients contemplated for laundry formulations.

A process for making alkyl sulphate solutions by mixing C₁₂-C₁₈ alkyl sulfuric acid and neutralizing with an organic amine and adding the mixture to an alkoxylated nonionic surfactant is disclosed in EP 609574.

According to the method of the present invention, m = 0 and R is a mixture of C₆-C₂₂ alkyls, preferably saturated or unsaturated, linear or branched C₈-C₁₈ having an even number of carbon atoms, wherein the amount of C8 and C₁₀ alkyls is from 1% to 10%. This feature is particularly advantageous over the prior art, since the presence of 1% - 10% of alcohols where R = C₈ and C₁₀ improves the fluidity performance of the product itself when m = 0. The use of non-ethoxylated alkyl sulphates is extremely important especially in North European countries where temperatures are lower and thus surfactants such as dodecylbenzenesulphonate biodegrade more slowly, and it is also essential in formulating Ecocert/Ecolabel products. Alkyl sulphates also improve cold wash performance.

In a preferred embodiment, the substantially anhydrous composition obtained with the method of the invention contains from 0 to 10% by weight of water, preferably from 4% to 6% by weight of water.

In another preferred embodiment, preferably the R group of the sulphuric acid ester of the formula R-(O-CH₂-CH₂)ₘ-OSO₃⁻H⁺ (see step b) of the method is a saturated or unsaturated, linear or branched C₈-C₁₈ alkyl having an even number of carbon atoms, or a mixture of saturated or unsaturated, linear or branched C₈-C₁₈ alkyls having an even number of carbon atoms.

The sulphuric acid ester R-(O-CH₂-CH₂)ₘ-OSO₃⁻H⁺ may be prepared by any of the sulfation methods per se known.

According to another preferred embodiment, preferably the R' group of the ethoxylated fatty alcohol R'-(O-CH₂-CH₂)ₓ-OH used as the solvent has a number of carbon atoms comprised between C₉-C₁₄, more preferably between C₁₀-C₁₃.

According to a further preferred embodiment, x is an integer comprised between 4 and 12, preferably between 4 and 8, more preferably between 6 and 8.

The organic amine base used for neutralization preferably is selected from the group consisting of triethanolamine, monoethanolamine, diethanolamine, monoisopropanolamine, triisopropanolamine, 2-aminobutanol, aminoethyl propanediol, arginine, lysine, ornithine, aminomethyl propanol, aminomethyl propanediol, 2-amino-2-hydroxymethyl-1,3-propanediol, triisopropanol and mixtures thereof. More preferred among them are monoethanolamine and monoisopropanolamine.

In the method of the invention, the ethoxylated fatty alcohol can be used as the solvent alone or in combination with another non aqueous solvent, for instance to improve the fluidity of the product. Preferably, the other non-aqueous solvent is selected from the group consisting of ethylene glycol, propylene glycol, dipropylene glycol, 1,3-butylene glycol, sorbitol, mannitol, erythritol, pentaerythritol and propanediol, glycerine, alkyl pentosides and mixtures thereof. A further preferred embodiment comprises adding urea, preferably in a concentration comprised between 0.01% and 10%, which helps to fluidize the product.

Also described, although not comprised within the scope of the invention, is a substantially anhydrous composition of alkyl (ethoxy) sulphates, which is neutralized with an organic amine base of the formula R-(O-CH₂-CH₂)ₘ-OSO₃⁻ BASE⁺ in an ethoxylated fatty alcohol of the formula R'-(O-CH₂-CH₂)ₓ-OH as the solvent, wherein R, m, R', x and the organic amine base are as previously defined with reference to the method of the invention, the composition containing from 0 to 10% by weight of water, preferably from 4% to 6% by weight of water.

In the present description, the term "neutralized alkyl (ethoxy) sulphate composition" is intended to mean a composition of neutralized alkyl sulphates and/or alkyl ethoxy sulphates.

The aforementioned composition is obtainable with the method of the invention. As previously indicated in the context of the method of preparation, the composition may comprise another non aqueous solvent as defined above and optionally urea, preferably in a concentration comprised between 0.01% and 10%.

Also described, although not comprised within the scope of the invention, is an ecodose, which is a washing machine detergent product comprising a pre-dosed amount of a liquid detergent for washing machine comprising the composition as previously described, obtained by the method of the invention, preferably in a concentration of from 30% to 90% by weight, more preferably from 60% to 80% by weight, the washing machine liquid detergent being contained in a sealed capsule made of a water soluble material. Such a water soluble material preferably is polyvinyl alcohol (PVA). The liquid detergent for washing machine may also comprise further conventional surfactants for these types of products, such as preferably soap and/or another ethoxylated fatty alcohol, as well as other conventional additives and/or excipients, such as for example optical bleaching agents, enzymes, anti-foaming agents, phosphonates, sequestrants, suspending agents, perfumes, colouring agents, and combinations thereof.

The examples that follow are provided solely by way of illustration and not of limitation of the scope of the invention as defined in the appended claims.

### EXAMPLES

### Example 1

Preparation of a MEA (monoethanolamine) alkyl sulphate solution in 7 moles ethoxylated branched C11 fatty alcohol.

125.66 g MEA were dissolved in 300 g of 7 moles of an ethoxylated branched C11 fatty alcohol and dropwise neutralised with 574.3 g of sulphuric acid ester R-O-SO₃⁻H⁺, where R has the following alkyl composition:
C8 = 4%
C10= 5.1%
C12 = 73.2%
C14 = 15.8%
C16 = 1.8%
C18 = 0.1%

The obtained product was left to homogenize. Given the prevalence of R = lauryl, for the sake of brevity the obtained product may be defined as a MEA lauryl sulphate solution in 7 moles ethoxylated branched C11 fatty alcohol. Such a product is a slightly clouded liquid with 53.2% of active ingredient. The appearance varies depending on pH: it is fluid and clear at a pH less than 8, whereas at a pH higher than 8, it tends to have a viscosity higher than 10000 cps at 20°C.

The obtained product is suitable for use in ecodoses.

### Example 2

Preparation of a MEA (monoethanolamine) alkyl sulphate solution in 7 moles ethoxylated branched C11 fatty alcohol mixture.

The same components, ratios and procedure of Example 1 were used, except that the alkyl R group of the sulphuric acid ester of the formula R-O-SO₃⁻H⁺ had the following alkyl composition:
C8 = 2%
C10= 7.1%
C12 = 72.3%
C14 = 14.3%
C16 = 4.1%
C18 = 0.2%

The obtained product is a clear liquid with 55.2% of active ingredient. Here also the appearance varies depending on pH: the product is fluid and clear at a pH less than 8, whereas at a pH higher than 8, it tends to have a viscosity higher than 10000 cps at 20°C.

The obtained product is suitable for use in ecodoses.

### Example 3

Preparation of a MEA (monoethanolamine) alkyl sulphate solution in 7 moles ethoxylated branched C11 fatty alcohol.

The same components, ratios and procedure of Example 1 were used, except that the alkyl R group of the sulphuric acid ester of the formula R-O-SO₃⁻H⁺ had the following alkyl composition:
C8 = 0.1%
C10 = 9.7%
C12 = 73.9%
C14 = 16%
C16 = 0.3%

The product is a slightly cloudy liquid with 57.3% of active ingredient. Such a product is a fluid liquid with a viscosity lower than 500 cps at 20°C and it is not affected by the pH. Thus, these are ideal conditions. We consider the above-indicated alkyl composition of the R chain the one with the best characteristics.

### Example 4

Preparation of a MIPA (monoisopropylamine) alkyl sulphate solution in 7 moles ethoxylated branched C11 fatty alcohol mixture.

In 150 g of propylene glycol and 150 g of 7 moles ethoxylated branched C11 fatty alcohol, 121.8 g MIPA were dissolved and dropwise neutralized with 574.3 g of sulphuric acid ester having the following formula: R-O-SO₃⁻H⁺, were R had the following alkyl composition:
C8 = 0.1%
C10 = 9.7%
C12 = 73.9%
C14 = 16%
C16 = 0.3%

The obtained product was left to homogenize. Here also, the obtained product has a prevalence of R = lauryl, so it may be defined as a MIPA lauryl sulphate solution in 7 moles ethoxylated branched C11 fatty alcohol. Such a product is a slightly clouded liquid with 48.9% of active ingredient. It appears as a fluid liquid with a viscosity below 200 cps at 20°C, and not affected by the pH. The reaction is more exothermic than Example 3 and the active ingredient is less, however the result is acceptable.

### Example 5

Preparation of a MEA (monoethanolamine) alkyl sulphate solution in 7 moles ethoxylated branched C11 fatty alcohol mixture.

The same method of Example 3 was carried out, though using a 50:50 mixture of plant-derived propylene glycol and 7 moles ethoxylated branched C11 fatty alcohol as the solvent. In short, the following ratios were used. In 150 g of propylene glycol and 150 g of 7 moles ethoxylated branched C11 fatty alcohol, 125.66 g MEA were dissolved and dropwise neutralized with 574.3 g of sulphuric acid ester having the following formula: R-O-SO₃⁻H⁺ where R has the following alkyl composition:
C8 = 0.1%
C10 = 9.7%
C12 = 73.9%
C14 = 16%
C16 = 0.3%

The obtained product was left to homogenize. The product is a clear liquid at 20°C with 61.5% of active ingredient. It appears as a fluid liquid with a viscosity below 200 cps at 20°C, and not affected by the pH. The use of propylene glycol as the solvent in combination with 7 moles of ethoxylated fatty alcohol improves the surfactant characteristics.

### Example 6

The same method of Example 3 was carried out, using 8 moles of ethoxylated linear C12 alcohol as the solvent and the following ratios, and modifying the chain so as to obtain a product that is more fluid.

In 300 g of 8 moles ethoxylated linear C12 alcohol, 125.66 g MEA were dissolved and dropwise neutralized with 574.3 g of sulphuric acid ester having the following formula: R-O-SO₃⁻ H⁺ where R has the following alkyl composition:
C8 = 0.1%
C10 = 9.7%
C12 = 73.9%
C14 = 16%
C16 = 0.3%

The obtained product was left to homogenize. The product is a clear liquid at 20°C with 55.3% of active ingredient, highly viscous at 20°C (8600 cps), not affected by the pH, but it becomes even more viscous at temperatures below 10°C.

### Example 7

Preparation of a MEA (monoethanolamine) alkyl ethoxy sulphate solution in 7 moles ethoxylated branched C11 fatty alcohol using 3 moles ethoxylated C12-C14 as the starting alcohol.

In 400 g of 7 moles ethoxylated branched C11 fatty alcohol, 125.66 g MEA were dissolved and dropwise neutralized with 840.5 g of sulphuric acid ester having the following formula: R-(OCH₂CH₂)₃O-SO₃⁻ H⁺ where R has the following alkyl composition:
3 moles ethoxylated C8 = 0.05%
3 moles ethoxylated C10 = 1.2%
3 moles ethoxylated C12 = 71.3%
3 moles ethoxylated C14 = 26.95%
3 moles ethoxylated C16 = 0.5%

The obtained product was left to homogenize. Such a product is a slightly cloudy liquid at 20°C with 60.1% of active ingredient. Its viscosity is 6300 cps at 20°C, and it is not affected by the pH.

### Example 8

Preparation of a MEA (monoethanolamine) alkyl ethoxy sulphate solution in 7 moles ethoxylated branched C11 fatty alcohol using 3 moles ethoxylated C12-C14 as the starting alcohol.

In 400 g of 7 moles ethoxylated branched C11 fatty alcohol, 125.66 g MEA were dissolved and dropwise neutralized with 840.5 g of sulphuric acid ester having the following formula: R-(OCH₂CH₂)₃O-SO₃⁻ H⁺ where R has the following alkyl composition:
3 moles ethoxylated C8 = 0.01%
3 moles ethoxylated C10 = 9.1%
3 moles ethoxylated C12 = 73.3%
3 moles ethoxylated C14 = 17.49%
3 moles ethoxylated C16 = 0.1%

The obtained product was left to homogenize. Such a product is a slightly clear liquid at 20°C with 60.9% of active ingredient. Its viscosity is 460 cps at 20°C, and it is not affected by the pH.

The best performance, also taking into consideration the better washing ability of alkyl sulphates, was obtained in Example 5. Anyway, all of the products manufactured as in the previous examples are suitable for use in the said ecodoses.

### Example 9

Three ecodoses according to the prior art currently on the market (i.e. Dash Ecodoses with Actilift, Selex Ecodoses, "Perle di Pulito Sole") were analysed and shown to be made up of the following ingredients:

| | |
|---|---|
| MEA Dodecylbenzenesulphonate | 20% - 40% active ingredient |
| Ethoxylated fatty alcohol | 10% - 30% active ingredient |
| MEA soap | 10% - 20% active ingredient |
| Propylene glycol and/or glycerine and/or other solvents | up to 100% |
| Enzymes, optical bleaching agents, complexing agents, excipients | q.s. |
| Water | 10% |

The following formulation was prepared replacing MEA dodecylbenzenesulphonate with the product manufactured in Example 5, designated as "MEA Alkyl Sulphate":

| MEA Alkyl Sulphate (Example 5) | 45% (prepared in the same way) |
|---|---|
| Laureth-7 | 22% |
| MEA Cocoate | 12.3% |
| Glycerine | 5 % |
| Propylene glycol | 12% |
| Sodium sulphite | 0.05% |
| Protease | 0.15% |
| Amylase | 0.15% |
| 1-Hydroxyethylidene-1,1-diphosphonic acid | 1% |
| Water | up to 100% |

The formulation of the invention was tested by comparison with the market leader and two medium-level benchmarks (i.e. Dash Ecodoses with Actilift, Selex Ecodoses, "Perle di Pulito Sole"), all containing MEA dodecylbenzenesulphonate.

The performance of the different formulations was assessed with a standard test by an external laboratory in accordance with the AISE 2009 protocol. The test conditions are described in Table 1.

**TABLE 1:**

| | |
|---|---|
| Dosage | 34 g/cycle |
| Wash program | Normal, without prewash |
| Temperature of the water | 40°C |
| Number of cycles | 6 |
| Soil replicas | 1 internal |
| Water in main wash | 151 |
| Simulating clean load | 3 kg of white cotton normalized with 3 washes at 60°C with ECE detergent, without optical bleaching agent or oxidants |
| Simulating soil load | 4 sheets of SBL 2004 (about 32 g of soil ballast) |
| Washing machine | Aqualtis AQ7L 05 U |
| Hardness of the water | 25°F (Italy's average) |

The formulations were tested on the following stains:
1) Tea - EMPA 167 (beverage / bleachable)
2) Coffee - CFT BC2 (beverage / bleachable)
3) Fruit juice - CFT CS15 (beverage / bleachable)
4) Chocolate - CFT CS44 (food / enzymatic)
5) Grass - EMPA 164 (generic soil / bleachable, enzymatic)
6) Motor oil - EMPA 106 (grease, oil / greasy dirt, particulate)
7) Make up - CFT CS17 (cosmetics/ grease, particulate)
8) Tomato sauce - Warwick-Equest WE5TPWKC (food / bleachable)
9) Blood - Warwick-Equest WE5DASBWKC (generic soil / enzymatic)
10) Grass and mud - Warwick-Equest WE51ACBFWKC (generic soil / bleachable, enzymatic, particulate)
11) Homogenized carrot - Warwick-Equest WE51ACBFWKC (food / bleachable, enzymatic)
12) Red wine - Warwick-Equest WE5RWWKC (beverage / bleachable)
13) Mustard - Warwick-Equest WE5FSMWKC (food / bleachable, enzymatic)
14) Fried food - Warwick-Equest WE5HBGBKC (on blue cotton) (grease, oil / greasy dirt, enzymatic)

In order to assess the soil-removal ability, the light reflectance was estimated through a spectrophotometer by measuring the Y value of the Y, x, z colour coordinates. The spectrophotometer features are set out in Table 2.

**TABLE 2:**

| | |
|---|---|
| Reading geometry | d/8 |
| Normalized illuminator/CIE standard observer | D65/10° |
| UV component of incident light | excluded by UV restriction filter (cut off at 420 nm) |
| Specular component | excluded |
| Area of illumination/measure | Illumination diam 30mm/Measure diam 26mm (LAV) |
| Assessed value | Y component |

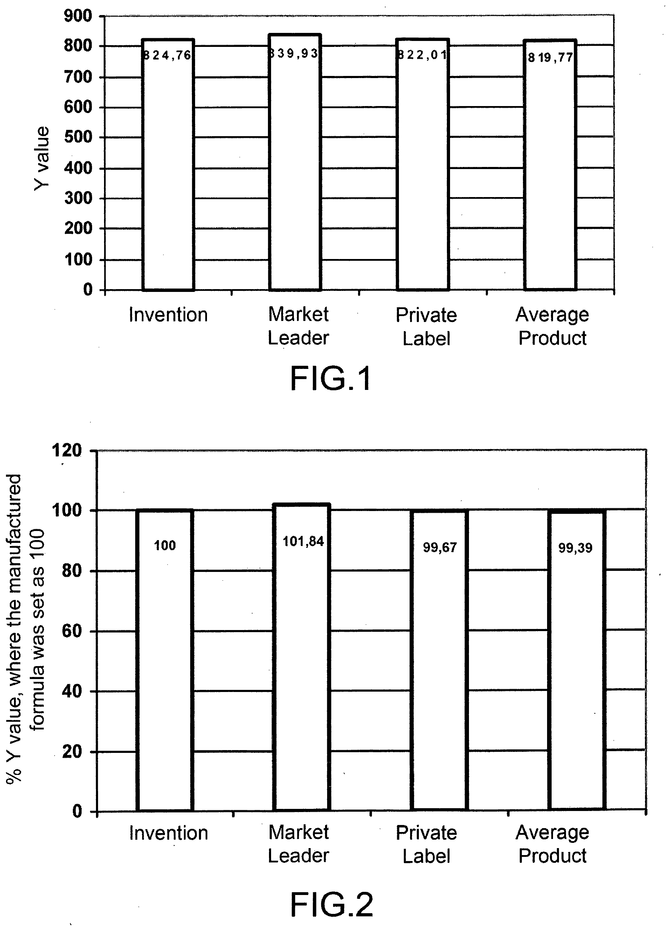

The stains were estimated twice, in the middle of the circular area of the stain itself. Higher the read values, better the performance. The results were assessed statistically (95% confidence limits). The appended figures 1 and 2 show the obtained results (Figure 1: test results, Y value; Figure 2: test results, % Y value). No statistically significant differences were observed among the samples.

## Claims

1. A method of preparing neutralized alkyl sulphates and/or alkyl ethoxy sulphates, comprising the steps of:
(a) mixing an ethoxylated fatty alcohol of formula:
R'-(O-CH₂-CH₂)ₓ-OH
wherein R' is a saturated or unsaturated, linear or branched C₆-C₁₆ alkyl, and x is an integer comprised between 1 and 12,
with an organic amine base; and
(b) adding to the mixture of the previous step a sulphuric acid ester of formula:
R-(O-CH₂-CH₂)ₘ-OSO₃⁻H⁺
wherein R is a mixture of saturated or unsaturated, linear or branched C₆-C₂₂ alkyls, wherein the amount of C₈ and C₁₀ alkyls is from 1% to 10%, and m is 0,
thereby obtaining a substantially anhydrous composition of alkyl (ethoxy) sulphate neutralised with an organic amine in an ethoxylated fatty alcohol as the solvent.

2. The method according to claim 1, wherein the substantially anhydrous composition contains from 0 to 10% by weight of water, preferably from 4 to 6% by weight of water.

3. The method according claim 1 or 2, wherein R is a mixture of saturated or unsaturated, linear or branched C₈-C₁₈ alkyls with an even number of carbon atoms, wherein the amount of C₈ and C₁₀ alkyls is from 1% to 10%.

4. The method according to any one of claims 1 to 3, wherein R' is a saturated or unsaturated, linear or branched C₉-C₁₄, preferably C₁₀-C₁₃ alkyl.

5. The method according to any one of claims 1 to 4, wherein x is an integer comprised between 4 and 12, preferably between 4 and 8, more preferably between 6 and 8.

6. The method according to any one of claims 1 to 5, wherein the organic amine base is selected from the group consisting of triethanolamine, monoethanolamine, diethanolamine, monoisopropanolamine, triisopropanolamine, 2-aminobutanol, aminoethyl propanediol, arginine, lysine, ornithine, aminomethyl propanol, aminomethyl propanediol, 2-amino-2-hydroxymethyl-1,3-propanediol, triisopropanol and mixtures thereof.

7. The method according to any one of claims 1 to 6, wherein in step (a) another non-aqueous solvent is added, selected from the group consisting of ethylene glycol, propylene glycol, dipropylene glycol, 1,3-butylene glycol, sorbitol, mannitol, erythritol, pentaerythritol and propanediol, glycerine, alkyl pentosides and mixtures thereof.

8. The method according to any one of claims 1 to 6, wherein in step (a) urea is added.

## Patentansprüche

1. Verfahren zum Herstellen von neutralisierten Alkylsulfaten und/oder Alkylethoxysulfaten, umfassend die Schritte:
(a) Mischen eines ethoxylierten Fettalkohols der Formel:
R'-(O-CH₂-CH₂)ₓ-OH
wobei R' ein gesättigter oder ungesättigter, linearer oder verzweigter C₆-C₁₆-Alkylrest ist, und x eine ganze Zahl zwischen 1 und 12 ist,
mit einer organischen Aminbase; und
(b) Hinzugeben zu der Mischung des vorherigen Schrittes eines Schwefelsäureesters der Formel:
R-(-O-CH₂-CH₂)ₘ-OSO₃⁻H⁺
wobei R eine Mischung aus gesättigten oder ungesättigten, linearen oder verzweigten C₆-C₂₂ Alkylresten ist, wobei die Menge an C₈ und C₁₀ Alkylresten 1 % bis 10 % beträgt, und m 0 ist,
und dadurch Erhalten einer im Wesentlichen wasserfreien Zusammensetzung aus Alkyl- (ethoxy) -sulfat neutralisiert mit einem organischen Amin in einem ethoxylierten Fettalkohol als das Lösungsmittel.

2. Verfahren nach Anspruch 1, wobei die im Wesentlichen wasserfreie Zusammensetzung von 0 bis 10 Gew.-% an Wasser enthält, bevorzugt von 4 bis 6 Gew.-% an Wasser.

3. Verfahren nach Anspruch 1 oder 2, wobei R eine Mischung aus gesättigten oder ungesättigten, linearen oder verzweigten C₈-C₁₈ Alkylresten mit einer geraden Anzahl an Kohlenstoffatomen ist, wobei die Menge an C₈ und C₁₀ Alkylresten 1 % bis 10 % beträgt.

4. Verfahren nach einem der Ansprüche 1 bis 3, wobei R' ein gesättigter oder ungesättigter, linearer oder verzweigter C₉-C₁₄ Alkylrest ist, bevorzugt ein C₁₀-C₁₃ Alkylrest.

5. Verfahren nach einem der Ansprüche 1 bis 4, wobei x eine ganze Zahl zwischen 4 und 12 ist, bevorzugt zwischen 4 und 8, noch weiter bevorzugt zwischen 6 und 8.

6. Verfahren nach einem der Ansprüche 1 bis 5, wobei die organische Aminbase ausgewählt ist aus der Gruppe bestehend aus Triethanolamin, Monoethanolamin, Diethanolamin, Monoisopropanolamin, Triisopropanolamin, 2-Aminobutanol, Aminoethylpropandiol, Arginin, Lysin, Ornithin, Aminomethylpropanol, Aminomethylpropandiol, 2-Amino-2-hydroxymethyl-1,3-propandiol, Triisopropanol, und Mischungen davon.

7. Verfahren nach einem der Ansprüche 1 bis 6, wobei in Schritt (a) ein anderes nichtwässriges Lösungsmittel hinzugefügt wird, das ausgewählt ist aus der Gruppe bestehend aus Ethylenglykol, Propylenglykol, Dipropylenglykol, 1,3-Butylenglykol, Sorbitol, Mannitol, Erythritol, Pentaerythrit und Propandiol, Glycerin, Alkylpentosiden und Mischungen davon.

8. Verfahren nach einem der Ansprüche 1 bis 6, wobei in Schritt (a) Harnstoff zugegeben wird.

## Revendications

1. Procédé de préparation d'alkyl sulfates et/ou d'alkyl éthoxy sulfates neutralisés, comprenant les étapes de :
(a) mélange d'un alcool gras éthoxylé de formule :
R'-(O-CH₂-CH₂)ₓ-OH
dans laquelle R' est un groupe alkyle en C₆-C₁₆ linéaire ou ramifié, saturé ou insaturé et x est un nombre entier compris entre 1 et 12,
avec une base amine organique ; et
(b) ajout au mélange de l'étape précédente d'un ester d'acide sulfurique de formule :
R-(O-CH₂-CH₂)ₘ-OSO₃⁻H⁺
dans laquelle R est un mélange de groupes alkyles en C₆-C₂₂ linéaires ou ramifiés, saturés ou insaturés, dans laquelle la quantité de groupes alkyles en C₈ et C₁₀ est de 1 % à 10 %, et m vaut 0,
permettant ainsi d'obtenir une composition substantiellement anhydre d'alkyl (éthoxy) sulfate neutralisé avec une amine organique dans un alcool gras éthoxylé en tant que solvant.

2. Procédé selon la revendication 1, dans lequel la composition substantiellement anhydre contient de 0 à 10 % en poids d'eau, de préférence de 4 à 6 % en poids d'eau.

3. Procédé selon la revendication 1 ou 2, dans lequel R est un mélange de groupes alkyles en C₈ à C₁₈ linéaires ou ramifiés, saturés ou insaturés, avec un nombre pair d'atomes de carbone, dans lequel la quantité de groupes alkyles en C₈ et C₁₀ est de 1 % à 10 %.

4. Procédé selon l'une quelconque des revendications 1 à 3, dans lequel R' est un groupe alkyle en C₉ à C₁₄, de préférence en C₁₀ à C₁₃, linéaire ou ramifié, saturé ou insaturé.

5. Procédé selon l'une quelconque des revendications 1 à 4, dans lequel x est un nombre entier compris entre 4 et 12, de préférence entre 4 et 8, de manière davantage préférée entre 6 et 8.

6. Procédé selon l'une quelconque des revendications 1 à 5, dans lequel la base amine organique est sélectionnée dans le groupe constitué de la triéthanolamine, de la monoéthanolamine, de la diéthanolamine, de la monoisopropanolamine, de la triisopropanolamine, du 2-aminobutanol, de l'aminoéthyl propanediol, de l'arginine, de la lysine, de l'ornithine, de l'aminométhyl propanol, de l'aminométhyl propanediol, du 2-amino-2-hydroxyméthyl-1,3-propanediol, du triisopropanol et de mélanges de ceux-ci.

7. Procédé selon l'une quelconque des revendications 1 à 6, dans lequel à l'étape (a) un autre solvant non aqueux est ajouté, sélectionné dans le groupe constitué de l'éthylène glycol, du propylène glycol, du dipropylène glycol, du 1,3-butylène glycol, du sorbitol, du mannitol, de l'érythritol, du pentaérythritol et du propanediol, de la glycérine, des alkyl pentosides et des mélanges de ceux-ci.

8. Procédé selon l'une quelconque des revendications 1 à 6, dans lequel à l'étape (a) de l'urée est ajoutée.
